# FASCICULE DE BREVET EUROPEEN

(11) **EP 2 528 638 B1**
(45) Date de publication et mention de la délivrance du brevet: **22.01.2014**
(21) Numéro de dépôt: 10808939.2
(22) Date de dépôt: 30.12.2010
(51) Int. Cl.: A61M 1/12, A61M 1/10

(54) **POMPE CARDIAQUE AUTONOME, ET PROCEDE MIS EN OEUVRE DANS UNE TELLE POMPE**
AUTONOME HERZPUMPE UND IN SOLCH EINE PUMPE IMPLEMENTIERTES VERFAHREN
SELF-CONTAINED CARDIAC PUMP, AND METHOD IMPLEMENTED IN SUCH A PUMP

(30) Priorité: 28.01.2010 FR 1050557
(43) Date de publication de la demande: 05.12.2012
(73) Titulaire: Fineheart, 33000 Bordeaux (FR)
(72) Inventeur: GARRIGUE, Stéphane, F-33130 Begles (FR)
(74) Mandataire: Pontet Allano & Associes
(86) Numéro de dépôt international: PCT/FR2010/052940
(87) Numéro de publication internationale: WO 2011/092394

(56) Documents cités:
- EP-A1- 1 129 736
- WO-A1-2010/010407
- US-A1- 2005 107 657
- US-A1- 2006 036 127
- US-A1- 2008 004 485
- US-B1- 7 022 100

## Description

La présente invention se rapporte à une pompe cardiaque artificielle permettant de réguler le débit sanguin.

Le coeur est un muscle creux qui par sa contraction rythmique assure la progression du sang dans les vaisseaux. Il comporte quatre cavités. L'oreillette droite et l'oreillette gauche disposées dans la partie supérieure du coeur ; le ventricule droit et le ventricule gauche disposés dans la partie basse.

Le ventricule droit est destiné à recevoir le sang en provenance de l'oreillette droite puis de l'éjecter dans l'artère pulmonaire. Cela constitue un « petit circuit » permettant d'envoyer le sang vers les poumons pour une réoxygénation.

Le ventricule gauche récupère le sang oxygéné depuis les poumons via l'oreillette gauche puis l'éjecte vers l'aorte pour apporter de l'oxygène à l'ensemble des tissus de l'organisme. Il s'agit de la « grande circulation » dite circulation systémique.

L'insuffisance cardiaque (IC), incapacité progressive du coeur à fournir un débit sanguin nécessaire aux besoins métaboliques d'un individu dans la vie courante, est la seconde cause de mortalité dans les pays occidentaux. Le traitement de l'insuffisance cardiaque, qui consiste à augmenter le débit sanguin de façon adaptée aux besoins du patient, est peu efficace avec les techniques actuelles, et coûte extrêmement cher.

On connaît le document US2009/0024212 décrivant une pompe pour traiter l'insuffisance cardiaque due à l'inactivité des valves sigmoïdes du coeur. Cette pompe présente une forme allongée s'étendant depuis l'intérieur du ventricule gauche jusqu'à l'intérieur de l'aorte de façon à remplacer la fonction des valves.

On connait également le document US6217541 décrivant une pompe cardiaque qui est également insérée à travers l'aorte jusqu'à l'intérieur du ventricule. L'extrémité de la pompe aspire le sang contenu dans le ventricule gauche puis le transfert vers l'aorte via un canal souple solidaire de l'extrémité de la pompe et disposé à travers les valves.

Les pompes décrites ci-dessus nécessitant une installation extrêmement complexe, et ne sont pas prévues pour une utilisation permanente.

On connaît aussi le document US6234772 décrivant une pompe rotative implantable. Cette pompe est de type à entrainement magnétique et permet de forcer la circulation du sang en évitant toute zone stagnante. Ce document reste muet quant à une quelconque mise en place efficace de la pompe.

Le document WO2010/010407 décrit une pompe rotative d'assistance cardiaque projetant le sang depuis le ventricule gauche à travers la valve aortique. Cette pompe est fixée à travers la valve aortique avec attaches de fixation dans l'aorte et au niveau de l'apex ventriculaire. Le moteur électrique est situé dans le conduit passant à travers la valve aortique.

On connaît enfin le document US2005/0107657 décrivant une pompe d'assistance ventriculaire gauche (pompe sanguine à flux mélangé) avec un circuit dit « radial » d'admission du sang et un circuit dit « axial » d'éjection du sang grâce à un propulseur rotatif situé au centre du dispositif. La base est maintenue au sein de la cavité ventriculaire gauche par une tige semi-rigide à travers l'apex du ventricule, alors que le sommet du dispositif passe à travers la valve aortique avec modification ou suppression fonctionnelle de cette valve. Chirurgicalement, une sternotomie avec mise en place d'une circulation extra-corporelle sont nécessaires, car il faut inciser au niveau de la racine de l'aorte. Le moteur électrique se situe à l'intérieur du dispositif, donc au sein du ventricule gauche. Ce document divulgue par ailleurs une équation d'efficience optimale entre le diamètre de la pompe et le nombre de tours/min du propulseur (jusqu'à 11 000 tours/min). Le diamètre de la pompe est donné à ∼ de 20-22 mm.

La présente invention a pour but une nouvelle pompe cardiaque peu complexe à installer par rapport à la mise en place de systèmes actuels.

Un autre but de l'invention est une simplicité dans la maintenance d'une telle pompe qui est prévue pour une utilisation à long terme.

L'invention a encore pour but une pompe peu invasive dans le ventricule du coeur et présentant une stabilité dans son maintien.

On atteint au moins l'un des objectifs précités avec une pompe cardiaque comprenant :
- un impulseur destiné à être inséré en partie dans le ventricule systémique d'un coeur, à travers la paroi de ce coeur, cet impulseur étant doté :
   - d'une membrane d'étanchéité et de fixation destinée à être suturée sur la paroi externe du coeur, l'épicarde, de façon à solidariser l'impulseur avec la paroi du coeur,
   - d'un carter destiné à être disposé à l'intérieur du ventricule systémique de façon à aspirer puis refouler le sang dans la direction de valves sigmoïdes du ventricule systémique,
   - d'un moteur destiné à être disposé en partie à l'extérieur du ventricule systémique et relié au carter ;
- une unité de gestion comprenant une alimentation et une unité de commande de l'impulseur;
- une liaison filaire entre l'unité de gestion et l'impulseur.

Par ventricule systémique, on entend le ventricule dédié à la circulation sanguine pour alimenter le corps d'un patient en oxygène via l'aorte. En principe, ce rôle incombe au ventricule gauche, mais dans certaines situations pathologiques, ce rôle peut être joué par le ventricule droit.

Avec la pompe cardiaque selon l'invention, l'impulseur est solidement fixé à la paroi du coeur, le patient peut se déplacer activement sans risque de lésion. On agit directement sur le débit sanguin en contrôlant directement la circulation sanguine. La présente pompe s'adresse à tous les patients insuffisants cardiaques sans critères pré-requis.

Par ailleurs, la disposition du moteur en partie à l'extérieur du coeur, signifie que ce moteur est accessible depuis l'extérieur du ventricule, cela permet une maintenance simplifiée.

Avantageusement, l'impulseur peut être inséré et suturé sur la partie basse du coeur à proximité de l'apex du coeur. De préférence, le moteur est amovible de sorte qu'il peut être remplacé aisément en cas de panne.

L'impulseur selon l'invention est un impulseur bio compatible de différents types, par exemple de type rotatif ou projectionnel.

Selon une première variante de l'invention, l'impulseur est de type rotatif et comprend un arbre à hélice disposé dans le carter. Dans ce cas, l'écoulement de sang se fait par force centrifuge. La seconde variante peut être caractérisée par le fait que l'impulseur est de type rotatif et comprend un arbre à vis d'Archimède ou à « vis sans fin » disposé dans le carter. Dans ce cas, l'écoulement se fait par poussée longitudinale le long du carter.

De préférence, le carter est un cylindre longiligne dont la paroi latérale est ajourée de façon à permettre l'écoulement de sang aspiré, et dont l'axe de révolution est en direction de valves sigmoïdes correspondantes. Une telle disposition permet d'éjecter le sang en direction de valves sigmoïdes, mais permet également d'aspirer efficacement le sang provenant de l'oreillette systémique. Par oreillette systémique, on entend l'oreillette associée au ventricule systémique.

Selon l'invention, l'unité de gestion peut être disposée à l'extérieur du patient, mais elle est de préférence en interne, et avantageusement dans la région épigastrique, dans la partie supérieure de l'abdomen. Ainsi, contrairement à des systèmes de l'art antérieur, l'alimentation selon l'invention est de préférence implantée dans son ensemble sans extériorisation. Pour ce faire, cette alimentation peut comprendre au moins une batterie, et de préférence une batterie rechargeable ; la recharge de la batterie pouvant éventuellement se faire par transduction per-cutanée.

La pompe selon l'invention peut ainsi être totalement implantée et autonome.

Selon une caractéristique avantageuse de l'invention, la pompe peut comprendre en outre une sonde, dite sonde d'activité pour recueillir l'activité cardiaque de façon à synchroniser le fonctionnement de l'impulseur avec l'activité électrosystolique cardiaque ; cette sonde d'activité peut être connectée à la paroi du coeur et peut être reliée de façon filaire à l'unité de gestion. Cette configuration permet de synchroniser le fonctionnement de l'impulseur avec le rythme cardiaque.

Dans une configuration tout intégré, la sonde d'activité est reliée à l'unité de gestion via ladite liaison filaire. Dans ce cas, cette liaison filaire constitue la seule liaison entre l'unité de gestion et l'impulseur.

Selon un mode de réalisation avantageux de l'invention, la pompe cardiaque comprend une sonde de recueil d'activité cardiaque et de stimulation, dite sonde systémique, connectée à la paroi du ventricule systémique et apte à communiquer avec l'unité de gestion de façon filaire ou non, par télémétrie sans fil notamment. Cette sonde joue un double rôle de recueil de l'information cardiaque et de stimulation cardiaque pour contracter le muscle en réponse à une consigne provenant de l'unité gestion. Une seconde sonde de même type, dite sonde non systémique, peut être prévue connectée à la paroi du ventricule non systémique et apte à communiquer avec l'unité de gestion de façon filaire ou non, par télémétrie sans fil notamment. Dans ce cas, on peut commander ces deux sondes pour réaliser une stimulation bi-ventriculaire. Le fait de pouvoir stimuler le coeur permet d'associer une action directe de l'impulseur sur le débit sanguin et une action indirecte de contraction cardiaque. Le rythme cardiaque détecté par différentes sondes permet en outre de synchroniser le fonctionnement de l'impulseur avec l'activité cardiaque. En d'autres termes, l'impulseur est synchronisé sur l'activité systolique ventriculaire lorsqu'il est possible de recueillir une information sur l'activité cardiaque, ou peut fonctionner en mode continu.

On peut également envisager une autre sonde de recueil d'activité cardiaque et de stimulation, dite sonde oreillette, connectée à la paroi de l'oreillette systémique et apte à communiquer avec l'unité de gestion de façon à parfaire le système de recueil de l'activité cardiaque et de stimulation. La communication peut être filaire ou non, par télémétrie sans fil notamment.

Une sonde est autonome énergétiquement lorsqu'elle communique de façon sans fil avec l'unité de gestion

En complément notamment de ce qui précède, la pompe selon l'invention peut avantageusement comprendre une sonde de recueil d'activité cardiaque, de stimulation et de défibrillation, dite sonde de défibrillation, connectée à la paroi du coeur et reliée de façon filaire à l'unité de gestion l'unité de commande étant en outre configurée en tant que défibrillateur.

On peut autrement prévoir une unité de gestion connectée de façon non filaire à un défibrillateur. Ce dernier peut être un défibrillateur externe (cutané) ou non, notamment automatique implantable indépendant mais communiquant avec l'unité de gestion par onde radio notamment.

Selon un mode de réalisation avantageux de l'invention, on dispose en outre un second impulseur tel que décrit ci-dessus sur le ventricule non systémique et on le relie également à l'unité de gestion.

Selon un autre aspect de l'invention, il est proposé un procédé pour réguler le débit sanguin dans un coeur au moyen d'une pompe cardiaque tel que décrit ci-dessus. Selon l'invention, on régule le débit sanguin en contrôlant la vitesse et la durée de fonctionnement de la pompe à partir de lois de commande prédéterminées ou à partir d'une consigne d'asservissement relative à l'activité cardiaque. Avec la consigne d'asservissement, on contrôle le débit sanguin en temps réel.

Avantageusement, on élabore la consigne d'asservissement en recueillant l'activité cardiaque au moyen d'une sonde connectée à la paroi du coeur et reliée de façon filaire à l'unité de gestion. On peut également réguler le débit sanguin en stimulant le coeur au moyen d'au moins une sonde de stimulation connectée à la paroi du coeur et reliée de façon filaire à l'unité de gestion.

D'autres avantages et caractéristiques de l'invention apparaîtront à l'examen de la description détaillée d'un mode de mise en oeuvre nullement limitatif, et des dessins annexés, sur lesquels :
- La figure 1 est une vue schématique simplifiée d'une pompe cardiaque selon l'invention insérée dans le ventricule gauche d'un coeur, et
- La figure 2 est une vue schématique d'une pompe cardiaque selon l'invention équipée d'une pluralité de sondes ou électrodes épicardiques de façon à synchroniser efficacement la pompe cardiaque par rapport à l'activité cardiaque.

Bien que l'invention n'y soit pas limitée, on va maintenant décrire une pompe cardiaque implantée dans le ventricule gauche d'un coeur qui est par principe le ventricule systémique. Mais, l'invention peut s'appliquer de la même manière à un ventricule droit lorsque ce dernier est le ventricule systémique.

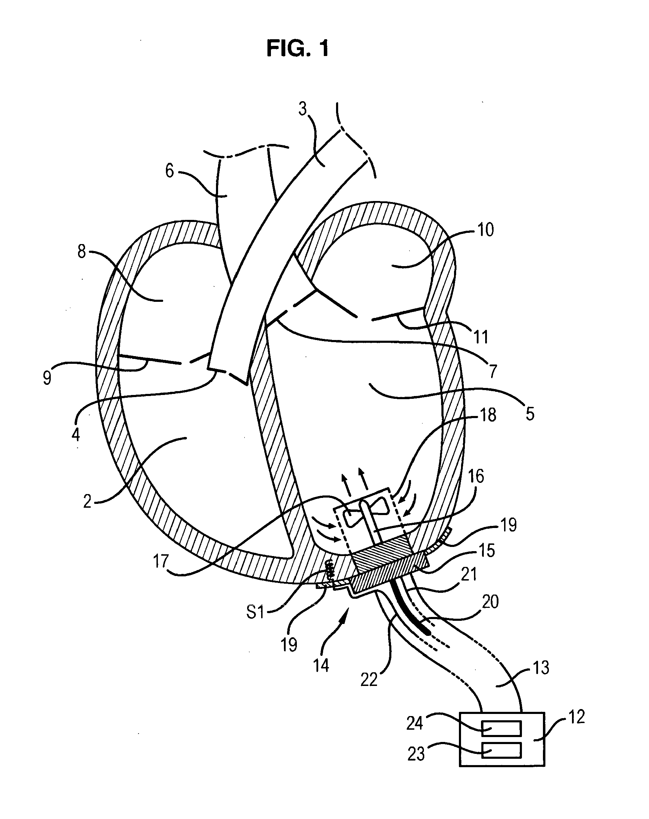

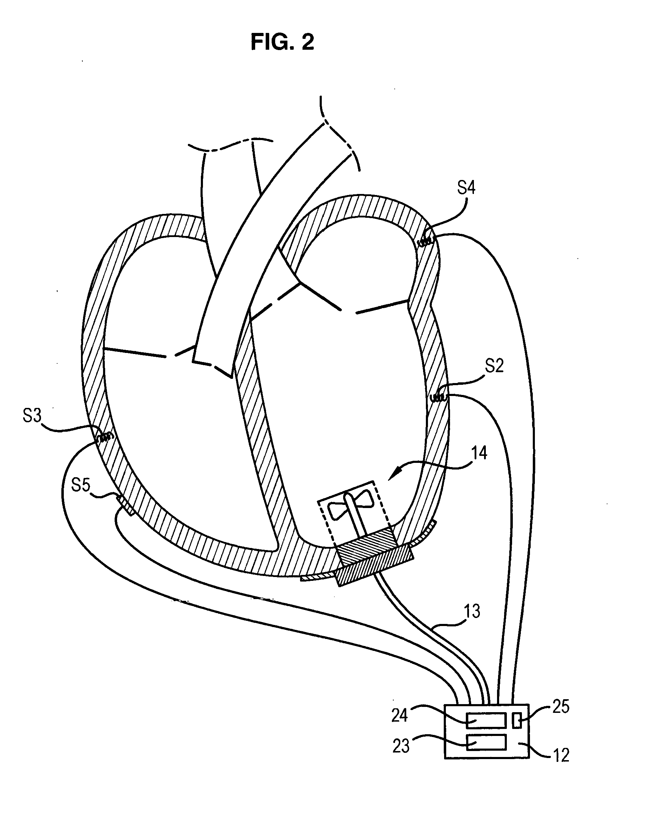

Sur les figures 1 et 2, les différents éléments communs aux diverses variantes ou formes de réalisation portent les mêmes références.

Sur les figures 1 et 2, le coeur est globalement désigné par la référence 1. On distingue le ventricule droit 2 qui a pour fonction d'éjecter le sang vers l'artère pulmonaire 3 à travers des valves sigmoïdes 4. Le ventricule gauche 5 a pour fonction de réaliser la circulation systémique en éjectant le sang rempli d'oxygène vers l'aorte 6 via des valves sigmoïdes 7.

L'oreillette droite 8 alimente le ventricule droit 2 en sang via des valves auriculo-pulmonaires 9. L'oreillette gauche 10 alimente le ventricule gauche 5 en sang via les valves auriculo-pulmonaires 11.

La pompe selon l'invention comporte une unité de gestion 12 reliée par une liaison filaire 13 à un impulseur 14 inséré en partie dans le ventricule gauche 5, au niveau de l'apex, c'est-à-dire à la pointe inférieure du ventricule gauche.

L'impulseur comporte un moteur 15 placé en grande partie à l'extérieur du ventricule gauche de sorte qu'il est facilement accessible à la suite d'une mini-thoracotomie (incision chirurgicale) et ou d'une opération par voie épigastrique en comparaison d'une sternotomie où l'on ouvre complètement le thorax. Ce moteur peut être un moteur à entraînement magnétique équipé d'un rotor s'étendant jusqu'à l'intérieur du ventricule sous la forme d'un arbre d'entrainement. L'arbre peut être de type « vis sans fin » (vis d'Archimède) permettant d'éjecter le sang depuis le fond du ventricule vers l'aorte 6. Cet arbre peut avantageusement être un arbre d'entrainement 16 avec une hélice 17 disposée à son extrémité libre. Cette hélice est conformée de telle sorte que la dynamique du fluide sanguin permet d'éjecter le sang vers l'aorte 6. Pour ce faire, un carter 18 de forme cylindrique est réalisé tout autour de l'arbre 16 et de son hélice 17. Ce carter 18 comporte au moins une ouverture, de préférence plusieurs ouvertures en nid d'abeille par exemple, sur sa paroi latérale de façon à permettre l'aspiration de sang provenant de l'oreillette gauche et son évacuation par l'ouverture supérieure du cylindre formant carter 18 par l'action de l'hélice 17. L'axe de rotation du carter cylindrique 18 est dirigé vers l'orifice aortique. Une telle orientation est avantageusement obtenue lors de la mise en place de l'impulseur par suturage. L'homme du métier comprendra aisément que d'autres types de moteurs miniaturisés biocompatibles peuvent être utilisés pour aspirer et refouler le sang. D'une façon générale les matériaux utilisés pour la mise en oeuvre de la pompe selon l'invention sont biocompatibles et peuvent donc être implantés dans le corps du patient.

L'impulseur 14 est inséré dans l'apex du coeur et y ait maintenu au moyen d'une membrane d'étanchéité et de fixation 19 de forme circulaire. D'autres types de membranes réalisant parfaitement l'étanchéité peuvent être envisagées. Cette membrane 19 est suturée sur la paroi externe du coeur tout autour du moteur 15 de façon à assurer une étanchéité totale entre le ventricule gauche 5 et l'extérieur du coeur.

La liaison filaire 13 relie l'impulseur 14 à l'unité de gestion 12 qui comporte une alimentation 23 telle qu'une batterie et une unité de commande 24 configurable à distance. La liaison filaire 13 comporte une ligne de commande 21 permettant à l'unité de commande 24 d'envoyer des consignes de commande vers l'impulseur 14, la ligne de commande 21 pouvant être bidirectionnelle. Le câble 20 est un câble d'alimentation du moteur 15. Le câble 22 permet de connecter électriquement l'unité de gestion 12 à une sonde d'activité S1 optionnelle insérée dans la paroi du coeur de façon à recueillir l'activité cardiaque du coeur. La sonde d'activité S1 peut être insérée à travers la membrane d'étanchéité et de fixation 19 ou bien au-delà afin de ne pas endommager l'étanchéité. Elle peut en outre être capable de stimuler le ventricule gauche ou droit. Dans ces cas, elle est disposée dans la paroi correspondant au ventricule gauche ou au ventricule droit.

Avec une telle pompe cardiaque selon l'invention, la liaison entre l'unité de gestion 12 et l'impulseur 14 est obtenue par l'unique liaison 13.

En fonctionnement, l'unité de gestion est configurée de façon à moduler la vitesse de rotation et la durée de fonctionnement du moteur en fonction de lois ou consignes de commande prédéterminées. Lorsqu'on dispose d'une sonde pour recueillir l'activité cardiaque comme par exemple la sonde d'activité S1, l'unité de commande 24 peut être configurée pour asservir le moteur au rythme cardiaque, en temps réel. Cet asservissement permet de synchroniser l'impulseur rotatif à la fréquence cardiaque.

De préférence, l'unité de gestion est implantée dans la région épigastrique, au sein de l'abdomen du patient. On peut ainsi prévoir que l'unité de commande 24 soit configurable à distance par communication sans fil.

Sur la figure 2, on voit un exemple de pompe cardiaque selon l'invention dans un mode de réalisation intégrant de nombreuses sondes ou électrodes épicardiques.

Les sondes disposées sur le coeur sont de types recueil d'information et stimulateur. Elles permettent d'identifier le début de l'activation électrique et de synchroniser l'impulseur sur l'ouverture des valves. Lorsque les deux ventricules sont soumis chacun à un impulseur, chaque impulseur est synchronisé à l'ouverture des valves correspondantes. Avantageusement, on adapte la fréquence de chaque impulseur de façon à délivrer un volume d'éjection systolique entre 20 et 35 ml pour chaque cycle cardiaque.

Étant donné que la mise en action d'un impulseur dans un ventricule à valve ouverte (durant la systole) augmente la quantité de sang éjectée, la pompe selon l'invention permet d'augmenter le volume d'éjection systolique et par conséquent, le débit sanguin.

Selon l'exemple illustré sur la figure 2, la pompe selon l'invention comporte une sonde de recueil d'activité cardiaque et de stimulation, dite sonde systémique S2 permettant notamment de stimuler le ventricule gauche par contraction musculaire. Cette sonde systémique S2, reliée à l'unité de gestion 12, est disposée dans la paroi du coeur au niveau du ventricule gauche. De la même manière une autre sonde de recueil d'activité cardiaque et de stimulation, dite sonde non systémique S3 est disposée sur la paroi du ventricule droit et est reliée à l'unité de gestion 12. Elle permet notamment de stimuler le ventricule droit par contraction musculaire. L'action combinée des deux sondes S2 et S3 permet de réaliser une stimulation bi-ventriculaire à partir de l'unité de commande 24 de façon à maintenir un rythme cardiaque selon une loi prédéterminée ou en réponse à des consignes données.

Sur la figure 2, on distingue également sur la paroi de l'oreillette gauche une sonde de recueil d'activité cardiaque et de stimulation, dite sonde oreillette S4, reliée à l'unité de gestion 12. Avantageusement, l'unité de commande 24 peut être configurée pour synchroniser la stimulation des sondes systémique S2 et non systémique S3 par rapport à l'information recueillie à partir de cette sonde S4.

En complément notamment de ce qui précède, chacune des sondes S2 et S4 peuvent jouer le rôle de la sonde d'activité S1.

Afin de traiter le risque de fibrillations ventriculaires, on prévoit un patch épicardique ou sonde de défibrillation S5 disposée sur la paroi externe du coeur, l'unité de commande étant configurée pour à la fois détecter une situation de fibrillation et de délivrer des impulsions électriques de faible énergie.

Pour appréhender complètement l'activité cardiaque, on prévoit un capteur d'activité 25 du patient, tel qu'un accéléromètre ou un capteur de pression, disposé par exemple dans l'unité de gestion 12 ou intégré à l'une des sondes précitées. Un tel capteur peut être utile pour un patient atteint d'une insuffisance chronotrope afin de détecter et signaler à l'unité de commande une quelconque accélération de l'activité physique du patient.

On prévoit également un capteur hémodynamique pour détecter l'état hémodynamique du patient de façon à compléter les informations obtenues sur le rythme cardiaque et commander efficacement l'impulseur. Le capteur hémodynamique peut être un capteur d'accélération endocardique de type PEA ( « Peak Endocardial Acceleration » en langue anglaise ) implanté par exemple ensemble avec l'électrode S2.

La pompe cardiaque selon l'invention permet donc de réguler le débit sanguin afin d'éviter toute insuffisance cardiaque. En outre, elle peut être implantée dans le coeur par mini-thoracotomie. L'impulseur rotatif peut être inséré au niveau de l'apex (la pointe inférieure) du ventricule gauche et si nécessaire un second impulseur rotatif peut être inséré à l'apex du ventricule droit. Ces deux impulseurs peuvent avantageusement être connectés à une unité de gestion placée dans la région épigastrique. Il s'agit ainsi d'un système fermé sans extériorisation de matériel électrique et d'alimentation.

## Revendications

1. Pompe cardiaque comprenant :
- un impulseur (14) destiné à être inséré en partie dans le ventricule systémique (5) d'un coeur, à travers la paroi de ce coeur, cet impulseur étant doté :
- d'une membrane d'étanchéité et de fixation (19) destinée à être suturée sur la paroi externe du coeur de façon à solidariser l'impulseur avec la paroi du coeur,
- d'un carter (18) destiné à être disposé à l'intérieur du ventricule systémique de façon à aspirer puis refouler le sang dans la direction de valves sigmoïdes (7) du ventricule systémique,
- d'un moteur (15) destiné à être disposé en partie à l'extérieur du ventricule systémique et relié au carter ;
- une unité de gestion (12) comprenant une alimentation (23) et une unité de commande (24) de l'impulseur; et
- une liaison filaire (13) entre l'unité de gestion et l'impulseur.

2. Pompe cardiaque selon la revendication 1, **caractérisée en ce que** la membrane d'étanchéité et de fixation (19) est de forme circulaire de façon à assurer une étanchéité totale et solidariser l'impulseur sur la partie basse du coeur à proximité de l'apex du coeur.

3. Pompe cardiaque selon la revendication 1 ou 2, **caractérisée en ce que** l'impulseur est de type rotatif et comprend un arbre (16) à hélice disposé dans le carter.

4. Pompe cardiaque selon la revendication 1 ou 2, **caractérisée en ce que** l'impulseur est de type rotatif et comprend un arbre à vis d'Archimède ou « vis sans fin » disposé dans le carter.

5. Pompe cardiaque selon l'une quelconque des revendications précédentes, **caractérisée en ce que** le carter (18) est un cylindre longiligne dont la paroi latérale est ajourée de façon à permettre l'écoulement de sang aspiré, et dont l'axe de révolution est en direction de valves sigmoïdes correspondantes.

6. Pompe cardiaque selon l'une quelconque des revendications précédentes, **caractérisée en ce que** l'unité de gestion (12) est biocompatible de sorte qu'elle est apte à être disposée à l'intérieur du patient dans la région épigastrique.

7. Pompe cardiaque selon l'une quelconque des revendications précédentes, **caractérisée en ce que** le moteur (15) est amovible.

8. Pompe cardiaque selon l'une quelconque des revendications précédentes, **caractérisée en ce que** l'alimentation (23) comprend au moins une batterie rechargeable.

9. Pompe cardiaque selon l'une quelconque des revendications précédentes, **caractérisé en ce qu'**elle comprend en outre une sonde, dite sonde d'activité (S1) pour recueillir l'activité cardiaque de façon à synchroniser le fonctionnement de l'impulseur avec l'activité électrosystolique cardiaque ; cette sonde d'activité étant connectée à la paroi du coeur et reliée de façon filaire à l'unité de gestion.

10. Pompe cardiaque selon l'une quelconque des revendications précédentes, **caractérisée en ce qu'**elle comprend une sonde de recueil d'activité cardiaque et de stimulation, dite sonde systémique (S2), connectée à la paroi du ventricule systémique et apte à communiquer avec l'unité de gestion.

11. Pompe cardiaque selon l'une quelconque des revendications précédentes, **caractérisée en ce qu'**elle comprend en outre une sonde de recueil d'activité cardiaque et de stimulation, dite sonde non systémique (S3), connectée à la paroi du ventricule non systémique et apte à communiquer avec l'unité de gestion.

12. Pompe cardiaque selon l'une quelconque des revendications précédentes, **caractérisée en ce qu'**elle comprend en outre une sonde de recueil d'activité cardiaque et de stimulation, dite sonde oreillette (S4), connectée à la paroi de l'oreillette systémique et apte à communiquer avec l'unité de gestion.

13. Pompe cardiaque selon l'une quelconque des revendications précédentes, **caractérisée en ce qu'**elle comprend en outre une sonde de recueil d'activité cardiaque, de stimulation et de défibrillation, dite sonde de défibrillation (S5), connectée à la paroi du coeur et reliée de façon filaire à l'unité de gestion ; l'unité de commande étant en outre configurée en tant que défibrillateur.

14. Pompe cardiaque selon l'une quelconque des revendications 1 à 12, **caractérisée en ce que** l'unité de gestion (12) est connectée de façon non filaire à un défibrillateur.

15. Pompe cardiaque selon l'une quelconque des revendications précédentes, **caractérisée en ce qu'**elle comprend en outre un second impulseur disposé sur le ventricule non systémique et relié à ladite unité de gestion.

## Patentansprüche

1. Herzpumpe, umfassend:
- einen Impulsgeber (14), der dazu bestimmt ist, teilweise in den systemischen Ventrikel (5) eines Herzens, durch die Wand dieses Herzens hindurch, eingesetzt zu werden, wobei dieser Impulsgeber ausgestattet ist mit:
- einer Dichtungs- und Fixiermembran (19), die dazu bestimmt ist, an der Außenwand des Herzens angenäht zu werden, um den Impulsgeber fest mit der Wand des Herzens zu verbinden,
- einem Gehäuse (18), das dazu bestimmt ist, innerhalb des systemischen Ventrikels angeordnet zu werden, um das Blut anzusaugen, anschließend in Richtung von Semilunarklappen (7) des systemischen Ventrikels auszustoßen,
- einem Motor (15), der dazu bestimmt ist, teilweise außerhalb des systemischen Ventrikels angeordnet und mit dem Gehäuse verbunden zu werden;
- eine Führungseinheit (12), die eine Versorgung (23) und eine Einheit zur Steuerung (24) des Impulsgebers umfasst; und
- eine kabelgebundene Verbindung (13) zwischen der Führungseinheit und dem Impulsgeber.

2. Herzpumpe nach Anspruch 1, **dadurch gekennzeichnet, dass** die Dichtungs- und Fixiermembran (19) eine Kreisform aufweist, um eine vollständige Dichtigkeit zu gewährleisten und um den Impulsgeber am unteren Teil des Herzens in der Nähe der Herzspitze fest zu verbinden.

3. Herzpumpe nach Anspruch 1 oder 2, **dadurch gekennzeichnet, dass** der Impulsgeber von rotierender Art ist und eine in dem Gehäuse angeordnete Welle (16) mit Propeller umfasst.

4. Herzpumpe nach Anspruch 1 oder 2, **dadurch gekennzeichnet, dass** der Impulsgeber von rotierender Art ist und eine in dem Gehäuse angeordnete archimedische Schrauben- oder "Schnecken"-Welle umfasst.

5. Herzpumpe nach einem der vorhergehenden Ansprüche, **dadurch gekennzeichnet, dass** das Gehäuse (18) ein schlanker Zylinder ist, dessen Seitenwand durchbrochen ist, um das Fließen von angesaugtem Blut zu ermöglichen, und dessen Rotationsachse in Richtung von entsprechenden Semilunarklappen ist.

6. Herzpumpe nach einem der vorhergehenden Ansprüche, **dadurch gekennzeichnet, dass** die Führungseinheit (12) biokompatibel ist, so dass sie geeignet ist, innerhalb des Patienten in der Oberbauchregion angeordnet zu werden.

7. Herzpumpe nach einem der vorhergehenden Ansprüche, **dadurch gekennzeichnet, dass** der Motor (15) lösbar ist.

8. Herzpumpe nach einem der vorhergehenden Ansprüche, **dadurch gekennzeichnet, dass** die Versorgung (23) wenigstens eine wiederaufladbare Batterie umfasst.

9. Herzpumpe nach einem der vorhergehenden Ansprüche, **dadurch gekennzeichnet, dass** sie ferner eine Sonde, sogenannte Aktivitätssonde (S1) zum Aufnehmen der Herzaktivität umfasst, um den Betrieb des Impulsgebers mit der elektrosystolischen Herzaktivität zu synchronisieren, wobei diese Aktivitätssonde an die Wand des Herzens angeschlossen und mit der Führungseinheit kabelgebunden verbunden ist.

10. Herzpumpe nach einem der vorhergehenden Ansprüche, **dadurch gekennzeichnet, dass** sie eine Sonde zum Aufnehmen der Herzaktivität und zur Stimulation, sogenannte systemische Sonde (S2) umfasst, die an die Wand des systemischen Ventrikels angeschlossen und geeignet ist, mit der Führungseinheit zu kommunizieren.

11. Herzpumpe nach einem der vorhergehenden Ansprüche, **dadurch gekennzeichnet, dass** sie ferner eine Sonde zum Aufnehmen der Herzaktivität und zur Stimulation, sogenannte nicht systemische Sonde (S3) umfasst, die an die Wand des nicht systemischen Ventrikels angeschlossen und geeignet ist, mit der Führungseinheit zu kommunizieren.

12. Herzpumpe nach einem der vorhergehenden Ansprüche, **dadurch gekennzeichnet, dass** sie ferner eine Sonde zum Aufnehmen der Herzaktivität und zur Stimulation, sogenannte Vorhofsonde (S4) umfasst, die an die Wand des systemischen Vorhofs angeschlossen und geeignet ist, mit der Führungseinheit zu kommunizieren.

13. Herzpumpe nach einem der vorhergehenden Ansprüche, **dadurch gekennzeichnet, dass** sie ferner eine Sonde zum Aufnehmen der Herzaktivität, zur Stimulation und zur Defibrillation, sogenannte Defibrillationssonde (S5) umfasst, die an die Wand des Herzens angeschlossen und mit der Führungseinheit kabelgebunden verbunden ist, wobei die Steuerungseinheit ferner als Defibrillator gestaltet ist.

14. Herzpumpe nach einem der Ansprüche 1 bis 12, **dadurch gekennzeichnet, dass** die Führungseinheit (12) mit einem Defibrillator kabellos verbunden ist.

15. Herzpumpe nach einem der vorhergehenden Ansprüche, **dadurch gekennzeichnet, dass** sie ferner einen zweiten Impulsgeber umfasst, der an dem nicht systemischen Ventrikel angeordnet und mit der Führungseinheit verbunden ist.

## Claims

1. Heart pump comprising:
- an impeller (14) intended to be inserted partly into the systemic ventricle (5) of a heart, through the wall of the heart, this impeller being equipped with:
- a sealing and fixing membrane (19) intended to be sutured to the outer wall of the heart in such a way as to secure the impeller to the wall of the heart,
- a housing (18) intended to be arranged inside the systemic ventricle in such a way as to draw up then discharge the blood in the direction of sigmoid valves (7) of the systemic ventricle,
- a motor (15) intended to be arranged partly outside the systemic ventricle and connected to the housing;
- a management unit (12) comprising a power supply (23) and an impeller control unit (24); and
- a wired link (13) between the management unit and the impeller.

2. Heart pump according to claim 1, **characterised in that** the sealing and fixing membrane (19) is circular in order to ensure a complete seal and secure the impeller to the bottom part of the heart near the apex of the heart.

3. Heart pump according to claim 1 or 2, **characterised in that** the impeller is of the rotative type and comprises a propeller shaft (16) arranged in the housing.

4. Heart pump according to claim 1 or 2, **characterised in that** the impeller is of the rotative type and comprises an Archimedes screw or "worn" shaft arranged in the housing.

5. Heart pump according to any one of the previous claims, **characterised in that** the housing (18) is a longilinear cylinder the side wall of which is perforated in such a way as to enable the discharge of blood drawn up, and the axis of rotation of which is facing corresponding sigmoid valves.

6. Heart pump according to any one of the previous claims, **characterised in that** the management unit (12) is biocompatible so that it is capable of being arranged inside the patient in the epigastric region.

7. Heart pump according to any one of the previous claims, **characterised in that** the motor (15) is removable.

8. Heart pump according to any one of the previous claims, **characterised in that** the power supply (23) comprises at least one rechargeable battery.

9. Heart pump according to any one of the previous claims, **characterised in that** it also comprises a sensor, called an activity sensor (S1), to collect cardiac activity in such a way as to synchronise the operation of the impeller with the electrosystolic activity of the heart; this activity sensor being connected to the wall of the heart and having a wired link to the management unit.

10. Heart pump according to any one of the previous claims, **characterised in that** it comprises a cardiac activity collection and stimulation sensor, called the systemic sensor (S2), connected to the wall of the systemic ventricle and capable of communicating with the management unit.

11. Heart pump according to any one of the previous claims, **characterised in that** it also comprises a cardiac activity collection and stimulation sensor, called the non-systemic sensor (S3), connected to the wall of the non-systemic ventricle and capable of communicating with the management unit.

12. Heart pump according to any one of the previous claims, **characterised in that** it also comprises a cardiac activity collection and stimulation sensor, called the atrium sensor (S4), connected to the wall of the systemic atrium and capable of communicating with the management unit.

13. Heart pump according to any one of the previous claims, **characterised in that** it also comprises a cardiac activity collection, stimulation and defibrillation sensor, called the defibrillation sensor (S5), connected to the wall of the heart and having a wired link to the management unit; the control unit also being configured as a defibrillator.

14. Heart pump according to any one of claims 1 to 12, **characterised in that** the management unit (12) has a wireless link to a defibrillator.

15. Heart pump according to any one of the previous claims, **characterised in that** it also comprises a second impeller arranged on the non-systemic ventricle and linked to said management unit.
